# FASCICULE DE BREVET EUROPEEN

(11) **EP 2 611 833 B1**
(45) Date de publication et mention de la délivrance du brevet: **26.06.2019**
(21) Numéro de dépôt: 11763995.5
(22) Date de dépôt: 30.08.2011
(51) Int. Cl.: C07K 16/26, C07K 16/28, A61K 39/395, A61P 35/00

(54) **ANTICORPS SE LIANT A L'ADRENOMEDULLINE ET AUX RECEPTEURS DE L'ADRENOMEDULLINE ET LEURS UTILISATIONS COMME MEDICAMENT**
ADRENOMEDULLIN UND ADRENOMEDULLIN-REZEPTOREN BINDENDE ANTIKÖRPER UND IHRE VERWENDUNG ALS WIRKSTOFFE
ANTIBODIES THAT BIND TO ADRENOMEDULLIN AND TO ADRENOMEDULLIN RECEPTORS AND THE USES THEREOF AS DRUGS

(30) Priorité: 30.08.2010 FR 1003472
(43) Date de publication de la demande: 10.07.2013
(73) Titulaire: Université d'Aix-Marseille, 13284 Marseille Cedex 07 (FR); Centre National de la Recherche Scientifique, 75016 Paris (FR)
(72) Inventeur: MABROUK, Kamel, 13170 Les Pennes Mirabeau (FR); OUAFIK, L'Houcine, 13005 Marseille (FR); TESIC, Carine, 13005 Marseille (FR); BERTIN, Denis, 13013 Marseille (FR); BERENGUER-DAIZE, Caroline, 13011 Marseille (FR); MARTIN, Pierre-Marie, 13008 Marseille (FR)
(74) Mandataire: Barbot, Willy
(86) Numéro de dépôt international: PCT/IB2011/053792
(87) Numéro de publication internationale: WO 2012/029023

(56) Documents cités:
- WO-A1-2010/012911
- WO-A2-02/066492
- WO-A2-2007/045927
- US-A1- 2007 004 630
- QI TAO ET AL: "Structure-function relationships of the N-terminus of receptor activity-modifying proteins.", BRITISH JOURNAL OF PHARMACOLOGY MAR 2010 LNKD- PUBMED:20015292, vol. 159, no. 5, mars 2010 (2010-03), pages 1059-1068, XP002638611, ISSN: 1476-5381
- Itidal Kaafarani ET AL: "Targeting adrenomedullin receptors with systemic delivery of neutralizing antibodies inhibits tumor angiogenesis and suppresses growth of human tumor xenografts in mice", The FASEB Journal, vol. 23, no. 10, 1 October 2009 (2009-10-01), pages 3424-3435, XP055442463, US ISSN: 0892-6638, DOI: 10.1096/fj.08-127852

## Description

La présente invention concerne le domaine des anticorps dirigés contre les récepteurs de l'adrénomédulline et utilisés comme médicament, destiné notamment au traitement des cancers.

L'adrénomédulline (AM) est un peptide vasoactif de 52 acides aminés (représenté en tant que séquence SEQ ID NO : 1) qui agit localement comme une hormone autocrine/paracrine et exerce de multiples actions biologiques (HINSON et al., Endocr Rev., 2000, 21 :138-67 ; CARON and SMITHIES, Proc Natl Acad Sci USA, 2001, 98:615-619 ; SHINDO et al., Circulation, 2001, 104: 1964-1971)..Des récepteurs de l'adrénomédulline sont présents non seulement dans les cellules de la plupart des tissus, tels que le coeur, le rein, le cerveau, le poumon et la glande surrénale, mais aussi dans les cellules du stroma des tumeurs.

L'adrénomédulline joue un rôle positif dans la régulation de l'angiogenèse lors du remodelage vasculaire en réponse à l'ischémie, dans l'appareil reproducteur de la femme durant le développement vasculaire embryonnaire, et lors du développement et de la vascularisation du placenta. Plusieurs équipes ont mis en évidence un rôle de ce peptide sur la prolifération, la migration et l'invasion des cellules endothéliales (OUAFIK et al., Am J Pathol, 2002, 160: 1279-92 ; KIM et al, FASEB J, 2003, 17: 1937-9 ; FERNANDEZ-SAUZE et al., Int J Cancer, 2004, 108:797-804). Il a aussi été montré que l'adrénomédulline agit sur une des dernières étapes de la néovascularisation qui consiste en la réorganisation des cellules endothéliales en tubules et ce, indépendamment du VEGF (FERNANDEZ-SAUZE *et al,.* 2004, cité ci-dessus).

Il a par ailleurs été mis en évidence un rôle de l'adrénomédulline dans la croissance tumorale (OUAFIK et al., Am J Pathol, 2002, 160: 1279-92 ; MARTINEZ et al., J Natl Cancer Inst., 2002, 94: 1226-37 ; OEHLER et al., Oncogene, 2001, 20:2937-45 ; ISHIKAWA et al., Oncogene, 2003, 22:1238-1242). Plusieurs études démontrent que l'adrénomédulline possède des propriétés angiogéniques dans la plupart des tumeurs (sein, prostate, colon, poumon, rein, voies respiratoires, vessie) (OUAFIK *et al.,* 2002, cité ci-dessus; FERNANDEZ-SAUZE *et al.,* 2004, cité ci-dessus ; NIKITENKO et al., Br J Cancer, 2006, 94: 1-7). Par exemple, chez des souris hétérozygotes adrénomédulline +/-, le volume tumoral diminue par rapport aux souris sauvages (IIMURO et al., Circ. Res., 2004, 95:415-423). Cet effet est associé à une réduction de la néo-vascularisation. Le blocage de l'action de l'adrénomédulline par un antagoniste (adrénomédulline₂₂₋₅₂) inhibe la croissance de tumeurs pancréatiques xénogreffées en déstabilisant la vascularisation des tumeurs (ISHIKAWA et al., Oncogene, 2003, 22:1238-1242). Des effets similaires ont été observés dans des xénogreffes développées à partir des cellules gliales tumorales (OUAFIK *et al.,* 2002, cité ci-dessus).

Les récepteurs de l'adrénomédulline (AMR) sont des complexes multiprotéiques composés de l'association d'au moins deux protéines, le CRLR («*Calcitonin Receptor Like Receptor* ») et une protéine RAMP (« *Receptor Activity-Modifying Protein* ») (McLATCHIE et al., Nature, 1998, 6683:333-9).

Le récepteur CLR (CRLR) a été isolé en 1993 (NJUKI et al.., Clin Sci, 1993 4:385-8 ; CHANG et al., Neuron., 1993, 6:1 187-95). Il comporte sept domaines transmembranaires couplés à une protéine G. La séquence peptidique du CLR fut établie chez l'Homme en 1996 (AIYAR et al., J Biol Chem., 1996, 19:1 1325-9) et chez le porc en 1998 (ELSHOURBAGY et al., Endocrinology, 1998, 4:1678-83). Le CLR appartient à la classe II des RCPG (« *G-protein-coupled receptors* »), classe qui regroupe des récepteurs de peptides tels que le glucagon et les peptides apparentés au glucagon (GLP), la sécrétine, la parathormone ou la calcitonine. Les RCPG sont de nature polypeptidique et comportent une partie extracellulaire portant le site de liaison du ligand, une partie transmembranaire à sept hélices et une partie intracellulaire en contact avec les protéines G qui assurent le transfert et l'amplification du signal reçu par le récepteur. Les RCPG présentent trois boucles extracellulaires (nommées El, E2 et E3) et trois boucles intracellulaires (11, 12 et 13) (BOCKAERT and PIN, Embo J, 1999, 18:1723-1729). Ces protéines peuvent être sujettes à des modifications post-traductionnelles, de type N-glycosylation, acétylation par des composés lipidiques formant parfois une pseudo-quatrième boucle intracellulaire (14) (ASSIE et al., EMC-Endocrinologie, 2004, 1 : 169-199).

En 1998, McLATCHIE et ses collaborateurs (référence citée ci-dessus) ont démontré que le récepteur CLR peut générer deux récepteurs pharmacologiquement distincts par association à une famille de protéines, de 160 acides aminés (14-21 kDa), à un seul domaine transmembranaire, nommées RAMPs. Le CLR n'est correctement fonctionnel qu'à l'état de dimère avec une protéine RAMP.

Il existe trois isoformes protéiques de RAMPs : RAMP1, 2 et 3. Ces protéines ont moins de 30% d'identité entre elles, mais présentent des similitudes d'organisation structurale. Chez l'Homme, les gènes codant RAMP1, RAMP2 et RAMP3 sont portés respectivement par les chromosomes 2, 17 et 7. Les protéines RAMPs sont constituées d'un seul domaine transmembranaire ; l'extrémité N-terminale extracellulaire est relativement longue et joue un rôle important dans la spécialisation et la fonctionnalité du récepteur (CGRP ou adrénomédulline) (KUWASAKO et al., J Biol Chem., 2001, 275:29602-9).

Deux fonctions essentielles sont attribuées aux protéines RAMPs :
- la détermination du récepteur : le rôle fondamental des protéines RAMPs est de définir la spécificité du ligand qui interagit directement à la surface cellulaire. RAMP1 présente le CLR comme une glycoprotéine mature pour former le récepteur du CGRP (« *calcitonin gene-related peptide* »). De même RAMP2 et RAMP3 présentent le CLR comme une glycoprotéine mature pour former les récepteurs de l'adrénomédulline. Ainsi la nature des protéines RAMPs présentes dans un type cellulaire, les interactions protéiques qui s'établissent entre les différents partenaires (CLR, RAMP1, RAMP2, RAMP3) et la proportion de chacune des protéines permettent aux cellules de répondre spécifiquement à différents neuropeptides (BÛHLMANN et al., FEBS Lett., 2000, 486:320-4 ; CHAKRAVARTY et al., Br J Pharmacol, 2000, 130:189-95) ;
- le transport intracellulaire : le CLR nécessite la co-expression des protéines RAMPs pour son transport vers la membrane cytoplasmique (SEXTON et al., Cell Signal, 2001 , 13:73-83). La réciproque est vraie : les protéines RAMPs ont besoin du CLR pour leur translocation à la surface cellulaire (FLAHAUT et al., J Biol Chem., 2002, 277: 14731-7).

Certains des Inventeurs ont déjà montré (Demande Internationale WO 2010/012911) qu'un mélange d'au moins trois anticorps se liant à trois protéines différentes formant les récepteurs de l'adrénomédulline, plus particulièrement les protéines hCLR, hRAMP2 et hRAMP3, présentait une efficacité anti-tumorale in vitro et in vivo significativement plus importante par rapport à l'utilisation d'un seul anticorps anti-CLR, anti-RAMP2 ou anti-RAMP3, ou même par rapport à l'utilisation d'un mélange de deux anticorps anti-CLR/anti-RAMP2 ou anti-CLR/anti-RAMP3.

Cependant, il existe toujours un besoin d'améliorer les traitements des maladies dans lesquelles l'angiogenèse doit être inhibée, notamment le cancer. En particulier, il existe un réel besoin de disposer de nouveaux anticorps (sérothérapie) ou d'antigènes (immunothérapie) pour lutter plus efficacement contre ces maladies.

Les Inventeurs ont maintenant synthétisé, un polypeptide chimère (dénommé aussi antigène) constitué de 4 séquences peptidiques dérivées respectivement de l'adrénomédulline et des récepteurs de l'adrénomédulline humains hRCLR, hRAMP2 et hRAMP3. Ils ont obtenu, chez le lapin, des anticorps polyclonaux dirigés contre ce polypeptide chimère, qui se lient à l'adrénomédulline, hCLR, hRAMP2 et hRAMP3. Ils ont alors montré que ces anticorps polyclonaux, antagonistes de l'adrénomédulline, hCLR, hRAMP2 et hRAMP3, présentaient une efficacité anti-tumorale *in vitro.* Ils ont aussi montré, *in vivo* chez la souris, que l'administration intra-péritonéale de ces anticorps anti-polypeptide chimère, d'une part induit une inhibition de la croissance tumorale des glioblastomes (U87) de 70% à 75% après 11 jours et jusqu'à 31 jours de traitement, et d'autre part inhibe le recrutement des cellules vasculaires (endothéliales et péricytaires). Plus particulièrement, les résultats obtenus dans le test d'angiogenèse in vivo démontrent la capacité de ces anticorps anti-polypeptide chimère à inhiber le recrutement des cellules vasculaires dès la première dose utilisée de 25 µg / souris, alors que l'équivalent de cette inhibition avec un mélange de trois anticorps se liant aux protéines hCLR, hRAMP2 et hRAMP3 n'est observé qu'avec une dose de 300 µg / souris, et que l'inhibition reste partielle avec un anticorps anti-adrénomédulline à une dose de 500 µg / souris.

Il ressort en outre de ces résultats qu'un tel polypeptide chimère (antigène) peut être utilisé pour la préparation d'un vaccin destiné au traitement d'une maladie dans laquelle l'angiogenèse doit être inhibée.

La présente invention a donc pour objet un polypeptide selon la revendication 1 ou la revendication 2.

Un polypeptide chimère (antigène) tel que défini ci-dessus, qui mime la structure des domaines extracellulaires des protéines CLR, RAMP2 et RAMP3, peut induire la production, chez les individus immunisés, d'anticorps antagonistes conformationnels qui reconnaissent spécifiquement ces protéines CLR, RAMP2 et RAMP3 sous leur forme native ou des complexes de protéines, c'est-à-dire les complexes AM/CLR, AM/RAMP2, AM/RAMP3, CLR/RAMP2, CLR/RAMP3, RAMP2/RAMP3, AM/CLR/R AMP2 , AM/CLR/RAMP3, AM/RAMP2/RAMP3 , CLR/RAMP2/RAMP3 ou AM/CLR/RAMP2/RAMP3.

Chacun des peptides suivants est utilisé pour obtenir un polypeptide chimère selon la présente invention :
- peptide dérivé de l'adrénomédulline, constitué de la concaténation des fragments R12-F14, R17-G19, K25-A27 et T34-S48 de l'adrénomédulline :
   RSFRFGKLATDKDKDNVAPRSKIS (SEQ ID NO : 2)
- peptides dérivés des fragments S27-K51 (SEQ ID NO : 3) ou P89-R119 (SEQ ID NO : 4) de la protéine hCLR :
   SPEDSIQLGVTRNKIMTAQYEAYQK (SEQ ID NO : 3),
   PDYFQDFDPSEKVTKIADQDGNWFRHPASNR (SEQ ID NO : 4),
- peptides dérivés des fragments K59-K81 (SEQ ID NO : 5) ou R91-R118 (SEQ ID NO : 6) de la protéine hRAMP2 :
   KNYETAVQFAWNHYKDQMDPIEK (SEQ ID NO : 5),
   RPYSTLRDALEHFAELFDLGFPNPLAER (SEQ ID NO : 6),
- peptides dérivés des fragments K49-K55 (SEQ ID NO : 7), L34-K55 (SEQ ID NO : 8) ou G91-E112 (SEQ ID NO : 9) de la protéine hRAMP3 :
   KVDVWK (SEQ ID NO : 7),
   LERLPLAGKAFADMMGKVDVWK (SEQ ID NO : 8),
   GFITGIHRQFFSNATVDRVHLE (SEQ ID NO : 9).

Le résidu d'acide aminé noté « A » correspond à un résidu alanine obtenu par substitution d'un résidu cystéine présent dans la séquence peptidique des protéines hCLR, hRAMP2 ou hRAMP3 naturelles. Le fait de substituer le résidu cystéine par une alanine permet d'obtenir une séquence peptidique linéaire bien caractérisée et évite d'obtenir des mélanges de peptides comprenant des dimères (par formation de ponts disulfures interchaînes).

L'ordre des différents peptides dérivés respectivement de l'adrénomédulline et des récepteurs de l'adrénomédulline CLR, RAMP2 et RAMP3 dans le polypeptide chimère selon la présente invention peut être aléatoire. Cependant, on préférera un polypeptide chimère comprenant ou constitué, de son extrémité N-terminale vers son extrémité C-terminale, d'un peptide dérivé de l'adrénomédulline, d'un peptide dérivé de CLR, d'un peptide dérivé de RAMP3 et d'un peptide dérivé de RAMP2, tels que définis ci-dessus.

Selon un mode de réalisation préféré de la présente invention, ledit polypeptide chimère (antigène) présente la séquence SEQ ID NO : 10 (dénommé ch2) : RSFRFGKLATDKDKDNVAPRSKISPDYFQDFDPSEKVTKIADQDGNWFRKVDVWK KNYETAVQFAWNHYKDQMDPIEK.

Le présent document décrit aussi un procédé d'obtention d'anticorps polyclonaux se liant à l'adrénomédulline ou aux protéines CLR, RAMP2 ou RAMP3, comprenant une étape d'immunisation d'un animal tel que défini ci-dessus avec un polypeptide chimère (antigène) tel que défini ci-dessus, de préférence le polypeptide chimère de séquence SEQ ID NO : 10.

La présente invention a également pour objet un polypeptide chimère (antigène) tel que défini ci-dessus, de préférence le polypeptide de séquence SEQ ID NO : 10, pour son utilisation comme médicament, de préférence comme vaccin, de préférence encore comme vaccin destiné au traitement préventif ou curatif d'une maladie dans laquelle l'angiogenèse doit être inhibée, consistant en le cancer, les maladies inflammatoires, le psoriasis, l'athérosclérose et la dégénérescence maculaire, de préférence le cancer.

Avantageusement, ledit vaccin est destiné au traitement préventif ou curatif de tumeurs, préférentiellement celles dont la vascularisation est nécessaire à leur croissance, plus particulièrement aux tumeurs solides.

Les antigènes selon l'invention sont préparés par les techniques classiques de synthèse en phase solide ou liquide, connues en elles-mêmes de l'homme du métier. Alternativement ils peuvent être préparés par les techniques d'ADN recombinant, connues aussi en elles-mêmes de l'homme du métier.

En conséquence, la présente invention a également pour objet une molécule d'acide nucléique isolée, comprenant une séquence codant pour un antigène (polypeptide chimère) tel que défini ci-dessus.

Les molécules d'acide nucléique selon l'invention sont obtenues par des méthodes classiques, connues en elles-mêmes de l'homme du métier, en suivant les protocoles standards.

La présente invention a également pour objet un vecteur recombinant eucaryote ou procaryote, comprenant un insert constitué par une molécule d'acide nucléique codant pour un antigène (polypeptide chimère) tel que défini ci-dessus. De nombreux vecteurs dans lesquels on peut insérer une molécule d'acide nucléique d'intérêt afin de l'introduire et de la maintenir dans une cellule hôte eucaryote ou procaryote, sont connus en eux-mêmes ; le choix d'un vecteur approprié dépend de l'utilisation envisagée pour ce vecteur (par exemple réplication de la séquence d'intérêt, expression de cette séquence, maintien de la séquence sous forme extra-chromosomique ou bien intégration dans le matériel chromosomique de l'hôte), ainsi que de la nature de la cellule hôte. Par exemple, on peut utiliser des vecteurs viraux ou non-viraux comme des plasmides.

De préférence, ledit vecteur recombinant est un vecteur d'expression dans lequel ladite molécule d'acide nucléique est placée sous le contrôle d'éléments régulateurs de la transcription et de la traduction appropriés. En outre, ledit vecteur peut comprendre des séquences (étiquettes ou tag) fusionnées en phase avec l'extrémité 5' et/ou 3' dudit insert, utiles pour l'immobilisation, et/ou la détection et/ou la purification de la protéine exprimée à partir dudit vecteur.

Ces vecteurs sont construits et introduits dans des cellules hôtes par les méthodes classiques d'ADN recombinant et de génie génétique, qui sont connues en elles-mêmes.

La présente invention a également pour objet des cellules eucaryotes ou procaryotes modifiées par un vecteur recombinant tel que défini ci-dessus.

Les vecteurs recombinants et les cellules transformées, tels que définis ci-dessus, sont utiles notamment pour la production de l'antigène tel que défini ci-dessus.

La présente invention a également pour objet une composition immunogène, caractérisée en ce qu'elle comprend un antigène (polypeptide chimère) tel que défini ci-dessus, éventuellement sous forme de micelles ou de vésicules de lipopeptide, associé à au moins un véhicule pharmaceutiquement acceptable et éventuellement à au moins un adjuvant.

Les adjuvants utilisés sont des adjuvants classiquement utilisés dans les compositions vaccinales, tels que l'hydroxyde d'alumine et le squalène.

D'autres aspects et avantages de la présente invention apparaîtront à la lecture des exemples qui suivent, qui doivent être considérés comme illustratifs et non limitatifs, ainsi que des figures annexées :

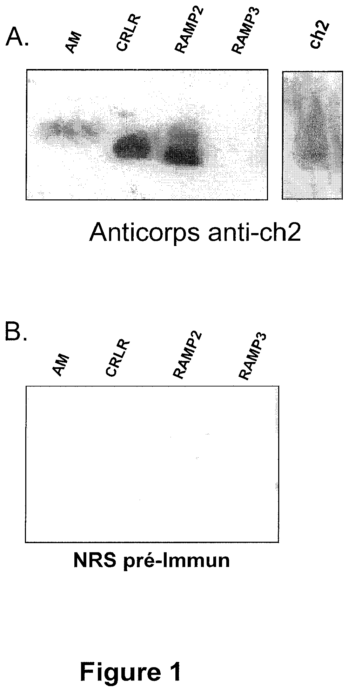

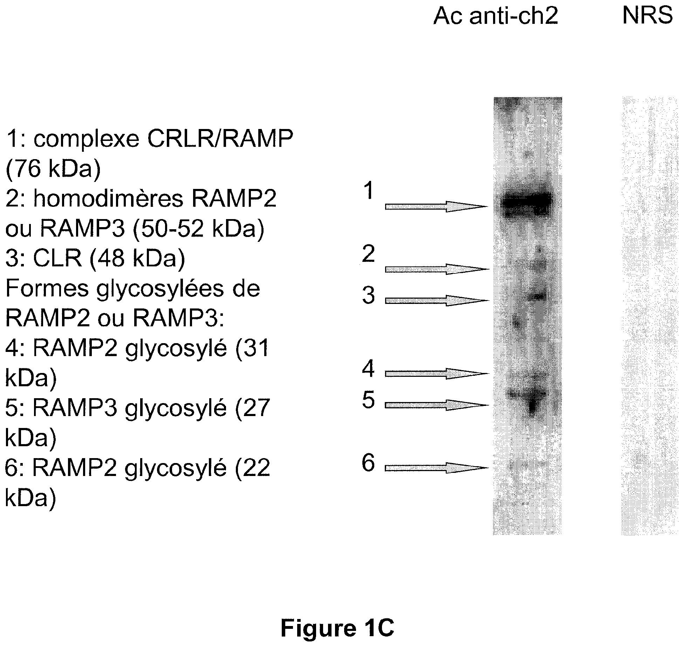

- **Figure 1** : Mise en évidence de la spécificité des anticorps polyclonaux anti-polypeptide chimère AM-CLR-RAMP3-RAMP2 (anticorps anti-ch2) par *Western Blot.* 750 ng de peptide/protéine adrénomédulline (AM) et hRAMP3, 500 ng de protéine hCLR (dénommé CRLR) et hRAMP2 et 20 ng de polypeptide chimère AM-CLR-RAMP3-RAMP2 (dénommé ch2) ont été séparés par SDS-PAGE sur gel d'acrylamide 17%, puis révélés avec les anticorps polyclonaux anti-polypeptide chimère AM-CLR-RAMP3-RAMP2 (Figure **1A**) ou avec le sérum non-réactif (NRS) (Figure **1B**)**,** utilisés au 1/250. La Figure **1C** représente la même expérience mais réalisée avec 20 µg d'extraits protéiques préparés à partir de cellules tumorales gliales U87, séparés par SDS-PAGE sur gel d'acrylamide 12%.

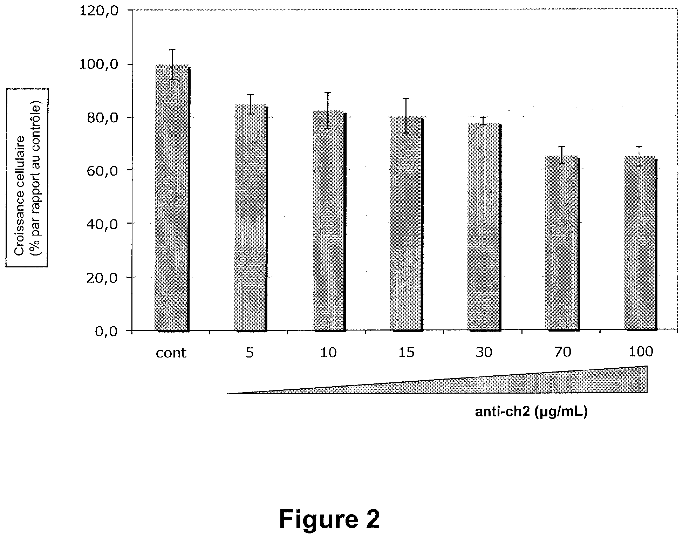

- **Figure 2** : Effet des anticorps polyclonaux anti-polypeptide chimère AM-CLR-RAMP3-RAMP2 (anti-ch2) sur la croissance des cellules tumorales gliales U87 in vitro : 2000 cellules par puits ont été ensemencées dans des plaques 24 puits, puis traitées pendant 6 jours avec les anticorps anti-polypeptide chimère AM-CLR-RAMP3-RAMP2 ou le sérum pré-immun (NRS) en milieu 2% sérum (2% de sérum foetal de boeuf dans 100 ml de milieu de culture). Un test de prolifération cellulaire MTT ([3,(4,5-dimethyl-thiazol-2-yl)-2,5-diphenyl tetrazolium bromide]) a ensuite été utilisé pour évaluer la quantité de cellules à la fin du traitement.

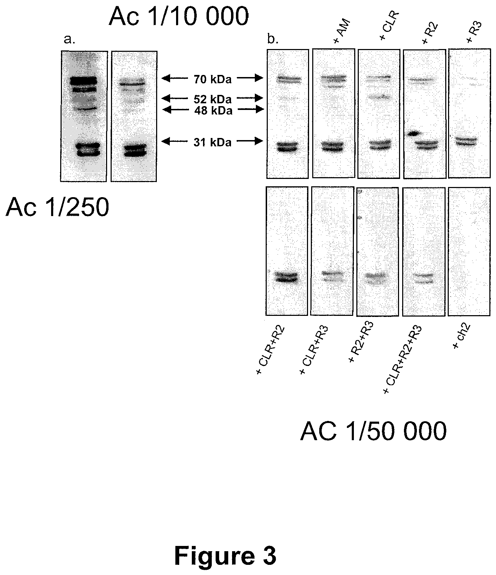

- **Figure 3** : *Western Blot* réalisé à partir d'extraits protéiques des cellules tumorales gliales U87. 20 µg de protéines totales ont été séparés par SDS-PAGE sur gel de polyacrylamide 12% puis révélées avec les anticorps polyclonaux anti-polypeptide chimère AM-CLR-RAMP3-RAMP2 utilisés au 1/250 ou au 1/10000 (Figure **3A**)**,** ou utilisés au 1/50000 (Figure **3B**) et pré-incubés 30 min avec 0,7 µg/mL d' adrénomédulline (AM), hCLR (CLR), hRAMP2 (R2) ou hRAMP3 (R3) ou 1,4 µg/mL de complexes protéiques hCLR/hRAMP2 (CLR+R2), hCLR/hRAMP3 (CLR+R3) ou hRAMP2/hRAMP3 (R2+R3) ou 2,1 µg/mL, de complexe protéique hCLR/hRAMP2/hRAMP3 (CLR+R2+R3) ou 0,7 µg/mL de polypeptide chimère AM-CLR-RAMP3-RAMP2 (ch2) (Figure **3B**)**.** La bande à 73 kDa correspond au complexe hCLR/hRAMP2 ou hCLR/hRAMP3, la bande à 50-52 kDa correspond aux homodimères hRAMP2 ou hRAMP3, la bande à 48 kDa correspond à hCLR et la bande à 31 kDa correspond à hRAMP2 glycosylée.

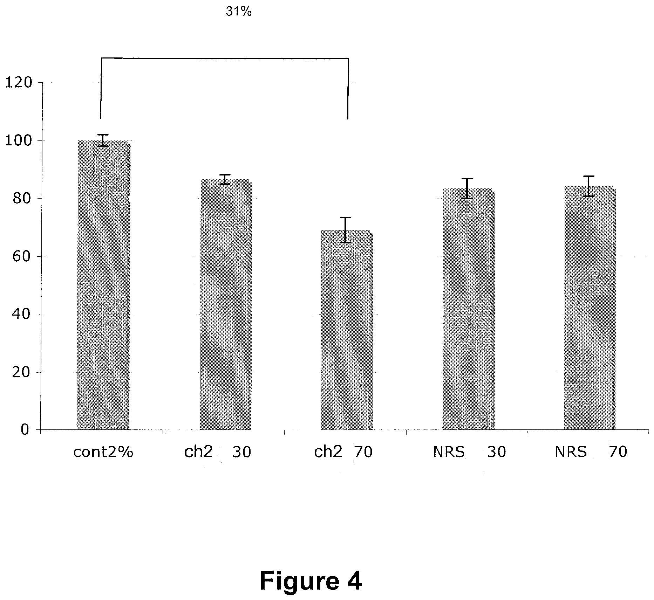

- **Figure 4** : Effet *in vitro* des anticorps polyclonaux anti-polypeptide chimère AM-CLR-RAMP3-RAMP2 sur la croissance des cellules U87. Des cellules tumorales gliales U87 ont été ensemencées dans des plaques de 24 puits (2000 cellules/puits) puis traitées pendant 6 jours avec les anticorps polyclonaux anti-polypeptide chimère AM-CLR-RAMP3-RAMP2 (dénommé ch2) à une concentration de 30 ou 70 mg/ml ou avec le sérum non-réactif (sérum pré-immun, NRS) à une concentration de 30 ou 70 mg/ml ou en milieu 2% sérum (cont2%). Un test MTT a ensuite été utilisé pour évaluer la quantité de cellules à la fin du traitement. Les chiffres « en

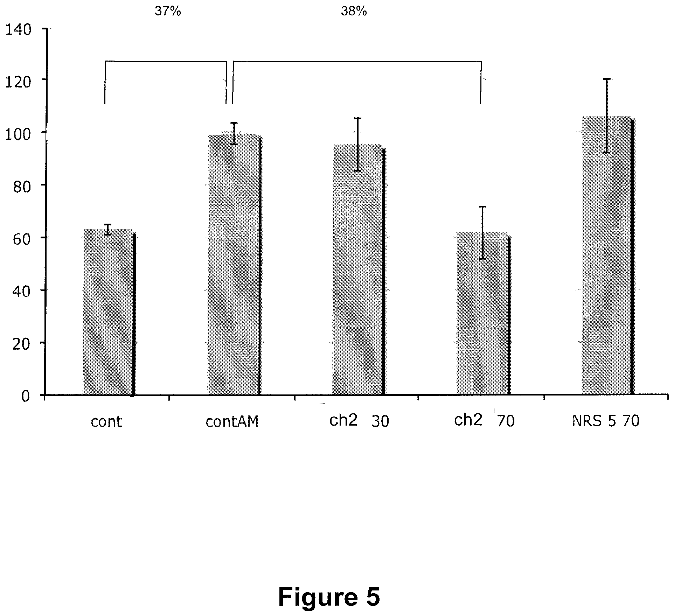

- **Figure 5** : Effet *in vitro* des anticorps polyclonaux anti-polypeptide chimère AM-CLR-RAMP3-RAMP2 sur la migration des cellules tumorales gliales U87. 20 000 cellules tumorales gliales U87 ont été ensemencées dans des chambres de Boyden en milieu 2% sérum après avoir été prétraitées (ch2) ou non (contAM) avec les anticorps polyclonaux anti-polypeptide chimère AM-CLR-RAMP3-RAMP2 ou avec le sérum non-réactif (NRS), puis ont été soumises pendant 4h à l'effet chimioattractant de l'adrénomédulline (10⁻⁷ M) située dans le compartiment inférieur de la chambre de Boyden. Un contrôle négatif a été effectué en omettant l'adrénomédulline dans le compartiment inférieur de la chambre de Boyden (cont). L'évaluation du nombre de cellules ayant migré a été effectué par comptage au microscope après un marquage au Dapi.

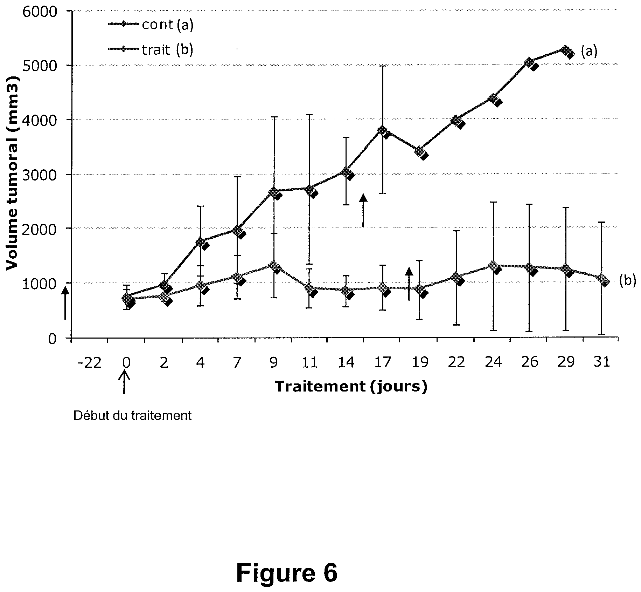

- **Figure 6** : Graphique montrant l'inhibition de la croissance tumorale des xénogreffes U87 chez la souris après traitement avec des anticorps anti-polypeptide chimère AM-CLR-RAMP3-RAMP2. 2 millions de cellules U87 ont été injectées à des souris par voie sous cutanée. 24 jours après l'injection, les tumeurs atteignant 600 à 800 mm³, les souris ont alors été séparées en deux groupes : le groupe contrôle recevant le sérum pré-immun NRS et le second groupe traité par voie intrapéritonéale avec les anticorps anti-polypeptide. chimère AM-CLR-RAMP3-RAMP2. Au bout de deux semaines de traitement à raison de 3 injections par semaine de 350 ug par injection d'anticorps, une diminution de 70% du volume tumoral a été observée.

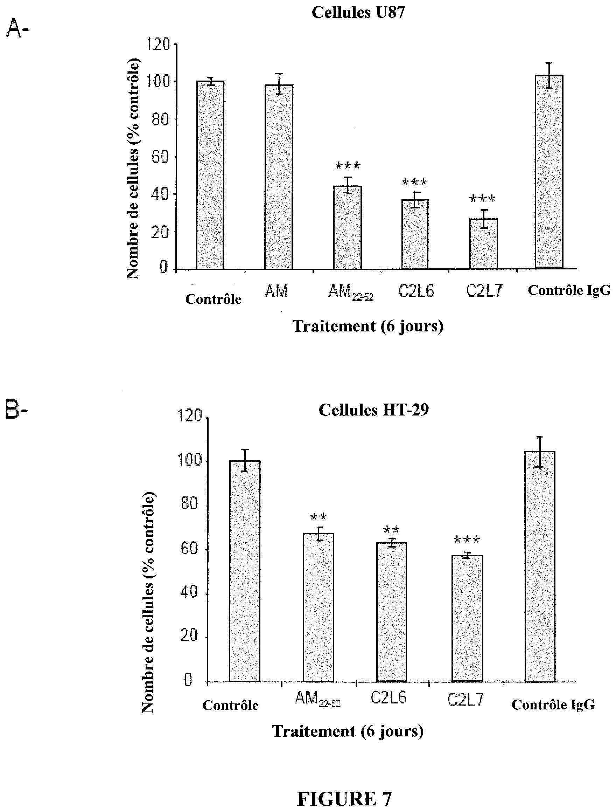

- **Figure 7** : Effet *in vitro* des anticorps polyclonaux anti-polypeptide chimère AM-CLR-RAMP3-RAMP2 sur la prolifération des cellules tumorales gliales U87 (A) et des cellules du cancer colorectal HT-29 (**B**)**.** Les cellules ont été traitées pendant 6 jours avec 2 lots d'anticorps polyclonaux anti-polypeptide chimère AM-CLR-RAMP3-RAMP2 différents (C2L6 et C2L7). Les contrôles sont: l'absence de traitement («Contrôle») ; traitement avec l'adrénomédulline (« AM ») ; traitement avec l'antagoniste AM₂₂₋₅₂ (« AM₂₂₋₅₂ ») ; traitement avec des IgG non spécifiques de AM, CLR, RAMP3 et RAMP2 (« Contrôle IgG »).

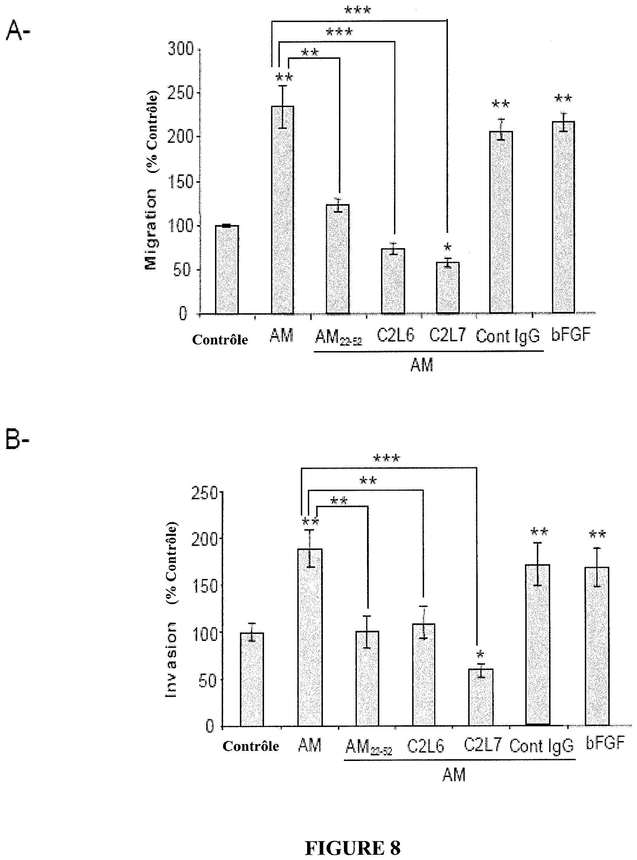

- **Figure 8** : Inhibition de la migration et de l'invasion des cellules U87 par les anticorps anti-polypeptide chimère AM-CLR-RAMP3-RAMP2, effectuée dans des chambres de Boyden. Les cellules ont été préalablement incubées ou non avec les anticorps anti-polypeptide chimère AM-CLR-RAMP3-RAMP2 (lots C2L6 ou C2L7) ou avec l'antagoniste AM₂₂₋₅₂. Les chemoattractants AM et bFGF ont été mis dans la chambre inférieure. Les contrôles sont: cellules sans traitement («Contrôle), cellules traitées avec l'adrénomédulline (AM), cellules traitées avec l'antagoniste AM₂₂₋₅₂ et pour lesquelles AM et bFGF ont été mis dans la chambre inférieure ; cellules traitées avec des IgG non spécifiques de AM, CLR, RAMP3 et RAMP2 et pour lesquelles AM et bFGF ont été mis dans la chambre inférieure (« Cont IgG ») ; cellules traitées avec des IgG non spécifiques de AM, CLR, RAMP3 et RAMP2.

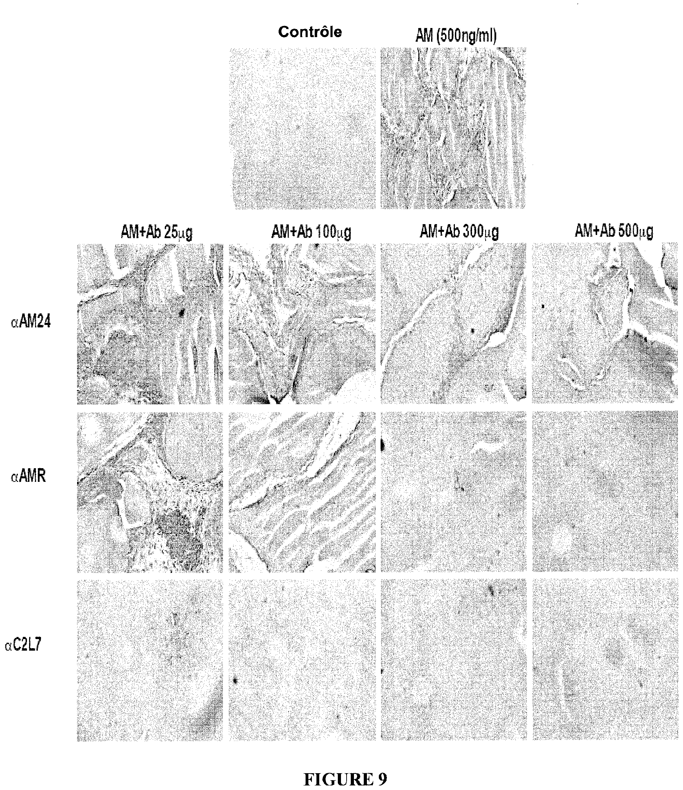

- **Figure 9** : Inhibition du recrutement des cellules vasculaires dans un test d'angiogenèse *in vivo.* Des souris C57BL/6 ont reçu par injection sous cutanée au niveau du l'aine 0,5 ml de Matrigel contenant ou pas (Contrôle) de l'AM (500 ng/ml). Les anticorps (Ab) anti-adrériomédulline (aAM₂₄), les anticorps (Ab) anti-récepteurs de l'adrénomédulline (anti-CLR, anti-RAMP2 et anti-RAMP3) (αAMR) ou les anticorps anti-polypeptide chimère AM-CLR-RAMP3-RAMP2 (lot C2L7) ont été administrés à raison de 25µg, 100 µg, 300 µg et 500 µg / souris par voie intra-péritonéale tous les 3 jours. Les traitements ont débuté 24 heures après l'injection du Matrigel. Après 15 jours de traitement, les souris ont été sacrifiées. Les Matrigels ont été isolés et fixés avec du formol, inclus dans la paraffine, puis coupés en section (6 µm) pour l'analyse avec l'hématoxyline et l'éosine.

### EXEMPLE

### I : MATERIEL ET METHODES

### I.1. Synthèse par voie chimique du polypeptide chimère de séquence SEQ ID NO : 10, dénommé polypeptide AM-CLR-RAMP3-RAMP2 (ou « ch2 »)

La synthèse du polypeptide de séquence SEQ ID NO : 10 en phase solide a été réalisée selon la méthode de Merrifield (MERRIFIELD, Science, 1986, 232:341-347). La méthode est basée sur l'utilisation d'un support solide (résine) sur lequel est ancré le polypeptide en voie d'élongation, et d'une phase liquide contenant les réactifs et solvants. L'assemblage du polypeptide est réalisé directement sur la matrice insoluble contenue dans un vase réactionnel, par incrémentation de la chaîne l'extrémité C-terminale jusqu'à l'extrémité N-terminale, suivant une série de réactions chimiques correspondant au cycle d'incorporation d'un acide aminé. Les divers acides aminés constitutifs du peptide sont donc couplés séquentiellement dans la chaîne polypeptidique suivant ce cycle répétitif, en fonction de la structure primaire de la molécule désirée. A chaque étape du cycle de synthèse, de simples filtrations et lavages permettent l'élimination des réactifs à travers la paroi filtrante du vase à réaction. En fin d'assemblage, un clivage chimique permet le "relargage" du polypeptide brut dans la phase liquide (MABROUK et al., Biochini. Biophys.Acta, 2007, 528:2528-2540).

Après purification, les peptides ont été caractérisés par spectrométrie de masse et par chromatographie liquide à haute pression (CLHP) analytique (colonne phase inversée C8).

### I.2. Obtention d'anticorps anti-polypeptide chimère AM-CLR-RAMP3-RAMP2

### Immunisations

Des anticorps polyclonaux ont été obtenus par injection, chez le lapin, du polypeptide chimère (antigène) AM-CLR-RAMP3-RAMP2 (ch2 ; SEQ ID NO : 10).

Les animaux ont été immunisés avec le polypeptide chimère AM-CLR-RAMP3-RAMP2 complété avec l'adjuvant de Freund. Des rappels d'immunisation ont ensuite été réalisés toutes les 3 semaines.

Les sérums non immuns (NRS), servant de contrôle (pré-immun), ont été prélevés chez les mêmes animaux avant l'immunisation.

### Purification des immunoglobulines (IgG) et dosage de l'endotoxine

Les anticorps polyclonaux ont été purifiés par passage sur gel de billes de sépharose couplées à la protéine A (GE Healthcare) et élués à l'aide de glycine 100 mM / pH 3. La présence d'endotoxine a été vérifiée à l'aide du test LAL (Limulus Amebocyte Lysate, Chambrex). Les résultats montrent un niveau tolérable de l'endotoxine (<1,25 U) dans les différentes préparations d'anticorps ainsi que dans le sérum pré-immun. Le calcul de la concentration en immunoglobuline a été effectué par la méthode de Pierce (Bicinchoninic (BCA) Protein Assays ; SMITH et al., Anal Biochem, 1985, 150:76-85).

Différents lots d'anticorps polyclonaux antagonistes anti-AM, -CLR, - RAMP3 et RAMP2 ont été obtenus.

### 1.3. Culture cellulaire

Les lignées cellulaires de glioblastome U87 (tumeur) proviennent de l'American Type Culture Collection (Rokville MD, USA); No ATCC : HTB-14. Les cellules sont maintenues dans un milieu approprié (milieu MEM + 2 mM glutamine + 1 mM sodium + pyruvate + 10% SVF) en atmosphère humide composée de 5% CO₂ et 95% air à 37°C.

Les lignées cellulaires de cancer colorectal HT-29 proviennent de l'American Type Culture Collection (Rokville MD, USA) ; No ATCC : HTB-38. Les cellules sont maintenues dans un milieu approprié (milieu DMEM + 2 mM glutamine + 10% sérum foetal de boeuf (FBS)).

Les milieux ont été renouvelés tous les deux jours. Lorsque les cellules ont atteint 90% de confluence, elles ont été détachées avec une solution de Trypsine (0,25%) en tampon Tris (Gibco) pendant quelques minutes à 37°C. L'action de l'enzyme a été arrêtée par addition de milieu contenant du sérum. Les cellules ont été ensemencées soit dans des tubes de 75 cm² soit dans des plaques multipuits dans leurs milieux appropriés.

### L4. Mise en évidence de la spécificité de liaison des anticorps anti-polypeptide chimère AM-CLR-RAMP3-RAMP2

a) 750 ng d'adrénomédulline (AM), 750 ng de protéine hRAMP3, 500 ng de protéine hCLR, 500 ng de protéine hRAMP2 et 20 ng de polypeptide chimère AM-CLR-RAMP3-RAMP2 ont été séparés par SDS-PAGE sur gel d'acrylamide 17%, puis révélés avec les anticorps anti-polypeptide chimère AM-CLR-RAMP3-RAMP2 ou avec le sérum non-réactif (NRS), utilisés au 1/250.
b) 20 µg d'extraits protéiques préparés à partir de cellules tumorales gliales U87 ont été séparés par SDS-PAGE sur gel d'acrylamide 12%, puis révélés avec les anticorps anti-polypeptide chimère AM-CLR-RAMP3-RAMP2 ou avec le sérum non-réactif (NRS), utilisés au 1/250.

### I.5.Etudes in vitro

### Etude in vitro de l'effet des anticorps polyclonaux anti-polypeptide chimère AM-CLR-RAMP3-RAMP2 sur la croissance des cellules tumorales gliales U87 et des cellules du cancer colorectal HT-29

Les cellules tumorales gliales U87 ou du cancer colorectal HT-29 ont été respectivement ensemencées dans des plaques de 24 puits (2000 cellules/puits) dans du milieu DMEM en présence de 2% de sérum (2% de sérum foetal de boeuf dans 100 ml de milieu de culture) puis traitées pendant 6 jours avec les anticorps polyclonaux anti-polypeptide chimère AM-CLR-RAMP3-RAMP2 ou avec le sérum non-réactif (NRS) en milieu 2% sérum. Un test MTT (OUAFIK et al., Am J Pathol., 2002, 160:1279-92; KAAFARANI et al., FASEB J., 2009, 23:3424-35) a ensuite été utilisé pour évaluer la quantité de cellules à la fin du traitement.

### Etudes in vitro de l'effet des anticorps polyclonaux anti-polypeptide chimère AM-CLR-RAMP3-RAMP2 sur la migration des cellules tumorales gliales U87

- *1^{ère}étude :* 20 000 cellules tumorales gliales U87 ont été ensemencées dans des chambres de Boyden en milieu 2% sérum après avoir été prétraitées ou non (contAM) avec les anticorps polyclonaux anti-polypeptide chimère AM-CLR-RAMP3-RAMP2 ou avec le sérum non-réactif (NRS), puis ont été soumises pendant 4h à l'effet chimioattractant de l'adrénomédulline (10⁻⁷ M) située dans le compartiment inférieur de la chambre de Boyden. Un contrôle négatif a été effectué en omettant l'adrénomédulline dans le compartiment inférieur de la chambre de Boyden (cont-). L'évaluation du nombre de cellules ayant migré a été effectuée par comptage au microscope après un marquage au Dapi (4',6'-diamidino-2-pheylindol).
- *2^{ème}étude :* Les tests de migration et invasion ont été effectués dans des chambres de Boyden, avec des filtres en polycarbonate possédant des pores de 8 µm de diamètre. Les filtres des cupules ont été incubés 1h à 37°C avec une solution de fibronectine (10 µg/ml, Sigma Aldrich) diluée dans du PBS pour la migration et avec une solution de Matrigel à 0,05 mg/ml pour l'invasion.

10 000 cellules U87 ont été déposées par cupule dans du milieu DMEM-2mM L-Glutamine-2% FBS. Les cellules ont été préalablement incubées ou non avec les anticorps anti-polypeptide chimère AM-CLR-RAMP3-RAMP2 (lots C2L6 ou C2L7) (70 µl/ml) ou avec l'antagoniste AM₂₂₋₅₂ (10⁻⁶M) (OUAFIK et al., Am J Pathol., 2002, 160:1279-92) pendant 30min à 37°C.

Les chemoattractants, AM (10⁻⁷M), bFGF (10⁻⁶M), ont été mis dans la chambre inférieure. Les plaques ont alors été placées à 37°C en atmosphère humide contenant 5% de CO2 pendant 2h. Une fixation à l'aide de Paraformaldehyde (PAF 4%) a été effectuée pendant 30 minutes à 37°C. La face supérieure du filtre, contenant les cellules qui n'ont pas migré, a été nettoyée délicatement avec un coton-tige après avoir été lavée au PBS. Sur la face inférieure, les cellules ayant migré ont été colorées au DAPI (Invitrogen Life Technologies) et le nombre de noyaux a été quantifié par le logiciel d'analyse d'images, Image J1.43u. Les valeurs représentant la moyenne ± SEM (« *Standard Error of the Mean* ») de 3 expériences indépendantes chacune a été réalisée en triplicate. (** *p <* 0,01 ; *** *p* < 0,001).

### I.6. Analyses par Western blot.

### Préparation des extraits protéiques

Les culots cellulaires provenant de cellules tumorales gliales U87 ou les homogénats obtenus à partir de tumeurs gliales xénogreffées chez les souris *nudes* ont été repris dans un tampon de lyse (20 mM HEPES pH 7,9, 10 mM NaCl, 1 mM MgCl₂, 10% glycérol, 0,2 mM EDTA, 0,5 mM DTT, 1% inhibiteurs des protéases et 0,35% de TritonX-100) et homogénéisés à 4°C. Après centrifugation à 12 000 x g pendant 10 minutes, le surnageant contenant les protéines a été récupéré et les protéines quantifiées par la méthode de Pierce.

### Western blot

Les lysats cellulaires (50 µg) ont été séparés par électrophorèse sur gel de polyacrylamide 12% en conditions dénaturantes et réductrices. A l'issue de la migration dans un tampon Tris-base 0,25 M, Glycine 1,92 M, SDS 1%, les protéines ont été transférées sur membrane de polyfluorure de vinylidène (PVDF) à 1 mA/cm2 pendant 1h30. Les membranes ont été saturées 1h à température ambiante dans du PBS-5% de lait écrémé. Après 2 lavages (PBS 0,2% - Tween 20), les membranes ont été incubées sous agitation pendant une nuit à 4°C en présence des anticorps anti-polypeptide chimère AM-CLR-RAMP3-RAMP2 dilués à 1/400 dans du PBS-1% lait écrémé. Après 3 lavages (PBS 0,2% - Tween 20), les membranes ont été incubées pendant 1h30 à température ambiante avec l'anticorps secondaire marqué à la peroxydase (ECL kit, GE Healthcare, Amersham). Le signal a été révélé en utilisant le kit de chimioluminescence (ECL kit, GE Healthcare, Amersham).

20 µg d'extraits protéiques préparés à partir de cellules tumorales gliales U87 ont été séparés par SDS-PAGE sur gel de polyacrylamide 12% puis révélées avec les anticorps polyclonaux anti-polypeptide chimère AM-CLR-RAMP3-RAMP2 utilisés au 1/250 ou au 1/10000, ou utilisés au 1/50000 et pré-incubés 30 min avec 0,7 µg/mL d'adrénomédulline (AM), hCLR, hRAMP2 ou hRAMP3 ou 1,4 µg/mL de complexes protéiques hCLR/hRAMP2, hCLR/hRAMP3 ou hRAMP2/hRAMP3 ou 2,1 µg/mL de complexe protéique hCLR/hRAMP2/hRAMP3 ou 0,7 µg/mL de polypeptide chimère AM-CLR-RAMP3-RAMP2.

### I.7. Etudes in vivo

### Modèles animaux

Des souris femelles Balb/C athymique (nu/nu) et des souris C57BL/6 (Harlan, France) âgées de 4-5 semaines ont été utilisées. Elles ont été maintenues dans des conditions stériles, à une température stable et ont reçu une alimentation adaptée. Les expériences *in vivo* ne commencent qu'après une période d'adaptation des animaux à leur nouvel environnement (10-15 jours après la réception).

### Développement des xénogreffes et traitements des animaux

Des cellules de la lignée tumorales U87 ont été injectées par voie sous-cutané dans le flanc de souris athymiques (nu/nu) à raison de 2,5x10⁶ cellules par animal. Les animaux ont été pesés régulièrement et le volume tumoral a été mesuré 3 fois par semaine et calculé selon la formule de l'ellipsoïde V= Longueur x largueur x épaisseur x 0,5236 mm³.

Lorsque les tumeurs ont atteint un volume tumoral de 500-1000 mm³ (12-15 jours après injection des cellules), les animaux ont été traités par voie intra-tumorale ou intrapéritonéale avec les anticorps anti-polypeptide chimère AM-CLR-RAMP3-RAMP2 (voir paragraphe 1.2 ci-dessus) dont la concentration finale était de 350 µg/animal et ce à raison de 3 injections/semaine. Les groupes d'animaux servant de contrôle ont été traités de la même manière mais avec un sérum pré-immun (NRS). Les volumes tumoraux ont été mesurés trois fois par semaine. Les valeurs sont la moyenne ± SE (« *standard error* ») de 5 souris/groupe.

Des animaux ont été sacrifiés à des temps variables au cours du traitement (j2, j7, j11, j16, et j21). Les tumeurs ont été immédiatement prélevées et fixées dans le formol. Elles ont ensuite été incluses dans de la paraffine pour les études immunohistochimiques.

Un groupe d'animaux traités pendant 21 jours, a reçu une injection de lectine biotinylée (Biotinylated-lycopersicon Esculentum (tomato) lectin, CliniSciences) sous anesthésie. Les animaux ont été perfusés avec une solution de paraformaldéhyde 4% qui permet la fixation des tissus *in vivo.* Les tumeurs et plusieurs organes (cerveau, poumon, coeur et rein) ont été prélevés et congelés dans l'azote liquide pour des études d'histologie et d'immunohistochimie.

### Inhibition du recrutement des cellules vasculaire dans un test d'angiogenèse in vivo

Des souris C57BL/6 ont reçu par injection sous cutanée au niveau du l'aine 0,5 ml de Matrigel contenant ou pas (Contrôle) de l'AM (500 ng/ml). Les anticorps anti-adrénomédulline (αAM₂₄), les anticorps anti-récepteurs de l'adrénomédulline (anti-CLR, anti-RAMP2 et anti-RAMP3) (αAMR) ou les anticorps anti-polypeptide chimère AM-CLR-RAMP3-RAMP2 (lot C2L7) ont été administrés à raison de 25µg, 100 µg, 300 µg et 500 µg / souris (n = 4) par voie intra-péritonéale tous les 3 jours. Les traitements ont débuté 24 heures après l'injection du Matrigel. Après 15 jours de traitement, les souris ont été sacrifiées. Les Matrigels ont été isolés et fixés avec du formol, inclus dans la paraffine, puis coupés en section (6 µm) pour l'analyse avec l'hématoxyline et l'éosine.

### Analyses en immunofluorescence

a) Les tumeurs de souris traitées avec 350 µg/animal d'anticorps anti-polypeptide chimère AM-CLR-RAMP3-RAMP2 ont été prélevées, fixées et incluses en parffine. Des coupes de 6 µm ont été réalisées sur microtome. Après déparaffinage et réhydratation, les lames ont subi un démasquage des épitopes par chauffage à 95°C dans un tampon Tris-EDTA (pH 9). Après traitement, les coupes ont été incubées pendant une nuit à 4°C avec l'anticorps anti-vWF (1/400 ; Dako), spécifique des cellules endothéliales. Le lendemain, les lames ont été lavées puis incubées avec l'anticorps secondaire peroxydase. La présence de l'anticorps est alors mise en évidence par une coloration au DAB. L'analyse du marquage a été réalisée à l'aide d'un microscope Leica équipé d'une lampe à fluorescence.
b) Les Matrigels contrôles, traités avec l'AM et ceux dont les souris ont été traitées avec 25 µg d'anticorps anti-polypeptide chimère AM-CLR-RAMP3-RAMP2 ont été analysés en *immunofluorescence* afin d'évaluer la neoangiogenèse. A cette fin, après réhydratation du Matrigel, le co-marquage avec l'anticorps anti-facteur VIII (vWF ; marqueur spécifique des cellules endothéliales) et l'anticorps anti-α-SMA (marqueur spécifique des cellules murales, les péricytes) a été réalisé. Les coupes ont été incubées pendant une nuit à 4°C avec les anticorps anti-α-SMA (1/80; Dako) et anti-vWF (1/100 ; Dako). Le lendemain, les lames ont été lavées puis incubées avec les anticorps couplés à un fluorochrome (Alexa 488 ou Alexa 568) pendant 2 heures à température ambiante dans le noir puis avec le DAPI (marqueur nucléaire ; 1/20000 d'une solution à 2mg/ml) (Invitrogen Lifes Technologies). Les lames ont été montées et l'analyse a été réalisée à l'aide d'un microscope Leica équipé d'une lampe à fluorescence.

### II : RESULTATS

### II.1. Mise en évidence de la spécificité des anticorps anti-polypeptide chimère AM-CLR-RAMP3-RAMP2

Les résultats du *Western-Blot* sont représentés à la Figure 1. Ces résultats montrent que les anticorps polyclonaux anti-polypeptide chimère AM-CLR-RAMP3-RAMP2 (dénommé ch2) se lient à l'adrénomédulline (AM), hCRL (CLR), hRAMP2 (R2) et hRAMP3 (R3), ainsi qu'aux complexes hCLR/hRAMP2, hCLR/hRAMP3, hRAMP2/hRAMP3 et hCLR/hRAMP2/hRAMP3, alors que les sérums non-réactifs (NRS) ne se lient pas à ces protéines ou complexes protéiques (voir Figures 1A et 1B).

### II.2. Efficacité anti-tumorale in vitro

L'analyse par la technique *Western-Blot* montre que les anticorps anti-polypeptide chimère AM-CLR-RAMP3-RAMP2 sont capables de reconnaître les peptides AM, RAMP2 et CLR. D'une façon très avantageuse ils reconnaissent les complexes formés par l'interaction CLR/RAMP2 et CLR/RAMP3 qui présentent un poids moléculaire de 73 kDa. Ces anticorps sont capables de reconnaitre, à la fois, la protéine CLR de 48 kDa, les homodimères RAMP2 ou RAMP3 présentant une taille de 50-52 kDa, deux formes monomériques RAMP2 glycolsylées présentant respectivement une taille de 22 ou 31 kDa et une forme monomérique RAMP3 glycosylée présentant une taille de 27 kDa (voir Figures 1C et 3).

L'incubation des cellules tumorales gliales U87 avec les anticorps anti-polypeptide chimère AM-CLR-RAMP3-RAMP2 démontre une inhibition de la prolifération de ces cellules d'une façon dose-dépendante (voir Figures 2 et 4). Ces expériences d'inhibition mettent aussi en évidence la présence d'une boucle autocrine et/ou paracrine faisant intervenir l'adrénomédulline et ses récepteurs CLR, RAMP2 et RAMP3 dans la prolifération des cellules tumorales.

De plus ces anticorps anti-polypeptide chimère AM-CLR-RAMP3-RAMP2 présentent une capacité à inhiber la migration des cellules U87 induite par l'AM d'une façon dose-dépendante (1^{ère} étude de migration ; voir Figure 5). Cependant, aucun effet d'inhibition n'a été observé sur les cellules pré-incubées avec le NRS (voir Figure 5).

### II.3. Inhibition de la prolifération des cellules de glioblastomes U87 et du cancer colorectal HT-29 par des anticorps anti-polypeptide chimère AM-CLR-RAMP3-RAMP2.

Les résultats sont représentés à la Figure 7. L'incubation des cellules U87 (A) et des cellules HT-29 (B) avec deux lots différents d'anticorps polyclonaux anti-polypeptide chimère AM-CLR-RAMP3-RAMP2 (lots C2L6 et C2L7) pendant 6 jours a provoqué l'inhibition de la prolifération. Cette diminution atteint 73 ± 4,5% pour les cellules U87 et 42,9 ± 1,1% pour les cellules HT-29 par rapport aux cellules contrôles et aux cellules traitées avec l'adrénomédulline exogène (AM ; SEQ ID NO : 1) exogène à 10⁻⁷ M. Le traitement avec l'antagoniste AM₂₂₋₅₂ à 10⁻⁶ M a été utilisé comme contrôle positif d'inhibition de la prolifération.

Ces résultats démontrent que les anticorps anti-polypeptide chimère AM-CLR-RAMP3-RAMP2 inhibent la boucle autocrine/paracrine impliquant l'AM et ses récepteurs (CLR/Ramp2 ; CLR/Ramp3) dans la croissance des cellules tumorales *in vitro.*

### II.4. Inhibition de la migration et l'invasion des cellules U87 par les anticorps anti-anti-polypeptide chimère AM-CLR-RAMP3-RAMP2 (2^{ème} étude de migration).

Les résultats sont représentés à la Figure 8. Ces résultats montrent la capacité des anticorps anti-polypeptide chimère AM-CLR-RAMP3-RAMP2 à inhiber la migration et l'invasion des cellules U87. Il est à noter que le lot C2L7 est capable d'inhiber d'une façon significative la migration et l'invasion stimulées par l'action endocrine de l'AM sur ses récepteurs par rapport aux contrôles.

### II.5. Efficacité anti-tumorale in vivo

Les tumeurs développées chez les souris athymiques après injection par voie sous-cutané de cellules de la lignée cellulaire U87 représentent un modèle expérimental qui prend en compte toutes les composantes du micro-environnement tumoral.

### Administration des anticorps par voie intra-péritonéale

L'administration par voie intra-péritonéale des anticorps anti-polypeptide chimère AM-CLR-RAMP3-RAMP2 induit une inhibition de la croissance tumorale des xénogreffes de 50% à 70% après 20 jours de traitement (voir Figure 6). Après 16 jours de traitement, il a été observé que les tumeurs d'animaux traités avec les anticorps anti-polypeptide chimère AM-CLR-RAMP3-RAMP2 apparaissaient pâles, translucides et. moins vascularisées. A l'opposé, les animaux contrôles présentaient des tumeurs larges très vascularisées. Ces effets importants observés *in vivo* laissent penser que, outre une action sur la prolifération des cellules tumorales, le traitement avec les anticorps anti-polypeptide chimère AM-CLR-RAMP3-RAMP2 perturbe un mécanisme fondamental indispensable à la croissance tumorale.

### Analyses en immunofluorescence

Les résultats montrent clairement une déstabilisation de la vascularisation intra-tumorale chez le groupe d'animaux traités avec les anticorps anti-polypeptide chimère AM-CLR-RAMP3-RAMP2 par rapport au groupe traité par les IgG contrôles.

### II.6. Inhibition du recrutement des cellules vasculaires dans le test d'angiogenèse in vivo

Les résultats sont représentés à la Figure 9. Ils montrent clairement la capacité des anticorps anti-polypeptide chimère AM-CLR-RAMP3-RAMP2 à inhiber le recrutement des cellules dès la première dose utilisée de 25µg / souris. L'équivalent de cette inhibition pour les αAMR n'est observé qu'avec des doses de 300 µg / souris, alors qu'avec l'αAM₂₄, l'inhibition reste partielle même à des doses de 500 µg / souris.

Ces résultats démontrent l'avantage et l'intérêt d'utiliser les anticorps anti-polypeptide chimère AM-CLR-RAMP3-RAMP2 pour inhiber de manière drastique l'effet de l'AM et ses récepteurs, par rapport à un anticorps anti-adrénomédulline ou à un mélange de 3 anticorps anti-récepteurs de l'adrénomédulline (anti-CLR, anti-RAMP2 et anti-RAMP3).

### Analyses en immunofluorescence

Les résultats montrent une vascularisation stable qui s'est mise en place dans le Matrigel contenant l'AM et qui persiste chez les souris traitées avec 25 µg d'anticorps αAM₂₄ et αAMR. De façon inattendue, on assiste à une disparition complète de cette vascularisation lors du traitement avec 25 µg d'anticorps anti-polypeptide chimère AM-CLR-RAMP3-RAMP2. On observe alors quelques cellules endothéliales sans la présence de cellules péricytaires.

Ces résultats suggèrent fortement la capacité des anticorps anti-polypeptide chimère AM-CLR-RAMP3-RAMP2 à inhiber le recrutement et le maintien des cellules vasculaires (endothéliales et péricytaires) dans le test d'angiogenèse *in vivo.* Le Matrigel contrôle montre quelques cellules détectées grâce au DAPI et qui sont de nature ni endothéliale ni péricytaire.

## Revendications

1. Un polypeptide chimère comprenant ou constitué de quatre peptides respectivement de l'adrénomédulline et d'un domaine extracellulaire des protéines CLR, RAMP2 et RAMP3, ledit polypeptide chimère étant capable d'induire, chez un animal immunisé avec ledit polypeptide chimère, la production d'anticorps polyclonaux se liant à l'adrénomédulline, CLR, RAMP2 et RAMP3 **caractérisé en ce que** le peptide de l'adrénomédulline présente la séquence SEQ ID NO : 2, le peptide de la CLR présente la séquence SEQ ID NO : 3 ou SEQ ID NO : 4, le peptide de la protéine RAMP2 présente la séquence SEQ ID NO : 5 ou SEQ ID NO : 6, et le peptide de la protéine RAMP3 présente la séquence SEQ ID NO : 7, SEQ ID NO : 8 ou SEQ ID NO :9.

2. Un polypeptide chimère capable d'induire, chez un animal immunisé avec ledit polypeptide chimère, la production d'anticorps polyclonaux se liant à l'adrénomédulline, CLR, RAMP2 et RAMP3, **caractérisé en ce qu'**il présente la séquence SEQ ID NO : 10.

3. Le polypeptide selon l'une quelconque des revendications 1 ou 2 pour son utilisation comme médicament.

4. Le polypeptide pour son utilisation selon la revendication 3, pour son utilisation comme vaccin destiné au traitement préventif ou curatif d'une maladie dans laquelle l'angiogenèse doit être inhibée, ladite maladie étant le cancer, les maladies inflammatoires, le psoriasis, l'athérosclérose ou la dégénérescence maculaire.

5. Une composition immunogène, **caractérisé en ce qu'**elle comprend un polypeptide chimère selon l'une quelconque des revendications 1 ou 2, associé à au moins un véhicule pharmaceutiquement acceptable et éventuellement à au moins un adjuvant.

6. Une molécule d'acide nucléique, **caractérisée en ce qu'**elle comprend une séquence codant pour un polypeptide chimère selon l'une quelconque des revendications 1 ou 2.

7. Un vecteur recombinant eucaryote ou procaryote, **caractérisé en ce qu'**il comprend un insert constitué par une molécule d'acide nucléique selon la revendication 6.

8. Cellules eucaryotes ou procaryotes modifiées par un vecteur recombinant selon la revendication 7.

## Patentansprüche

1. Chimäres Polypeptid, vier Peptide von Adrenomedullin bzw einer extrazellulären Domäne der Proteine CLR, RAMP2 und RAMP3 aufweisend oder aus ihnen bestehend, wobei das genannte chimäre Polypeptid in der Lage ist, bei einem mit dem genannten chimären Polypeptid immunisierten Tier die Erzeugung polyclonaler Antikörper zu induzieren, die an Adrenomedullin, CLR, RAMP2 und RAMP3 binden, **dadurch gekennzeichnet, dass** das Adrenomedullin-Peptid die Sequenz SEQ ID NO : 2 aufweist, das CLR-Peptid die Sequenz SEQ ID NO : 3 oder SEQ ID NO : 4 aufweist, das Peptid des Proteins RAMP2 die Sequenz SEQ ID NO : 5 oder SEQ ID NO : 6 aufweist, und das Peptid des Proteins RAMP3 die Sequenz SEQ ID NO : 7, SEQ ID NO : 8 oder SEQ ID NO : 9 aufweist.

2. Chimäres Polypeptid, in der Lage, bei einem mit dem genannten chimären Polypeptid immunisierten Tier die Erzeugung polyclonaler Antikörper zu induzieren, die an Adrenomedullin, CLR, RAMP2 und RAMP3 binden, **dadurch gekennzeichnet, dass** es die Sequenz SEQ ID NO : 10 aufweist.

3. Polypeptid nach irgendeinem der Patentansprüche 1 oder 2 zum Gebrauch als Medikament.

4. Polypeptid zum Gebrauch nach Patentanspruch 3 zum Gebrauch als Impfstoff zur vorbeugenden oder heilenden Behandlung einer Krankheit, in der die Angiogenese unterbunden werden muss, wobei die genannte Krankheit Krebs ist, Entzündungskrankheiten, Psoriasis, Atherosklerose oder Makuladegeneratio**n.**

5. Immunogene Zubereitung, **dadurch gekennzeichnet, dass** sie ein chimäres Polypeptid nach irgendeinem der Patentansprüche 1 oder 2 enthält, verbunden mit mindestens einem pharmazeutisch verträglichen Träger und gegebenenfalls mit mindestens einem Hilfsstoff.

6. Nukleinsäuremolekül, **dadurch gekennzeichnet, dass** es eine Sequenz enthält, die ein chimäres Polypeptid nach irgendeinem der Patentansprüche 1 oder 2 codiert.

7. Rekombinanter eukaryotischer oder prokaryotischer Vektor, **dadurch gekennzeichnet, dass** er ein Insert enthält, bestehend aus einem Nukleinsäuremolekül nach Patentanspruch 6.

8. Durch einen rekombinanten Vektor nach Patentanspruch 7 modifizierte, eukaryotische oder prokaryotische Zellen.

## Claims

1. A chimeric polypeptide comprising or consisting of four peptides from adrenomeddulin and from an extracellular domain of the CLR, RAMP2 and RAMP3 proteins respectively, wherein said chimeric polypeptide induces the production of polyclonal antibodies which bind to adrenomedullin, CLR, RAMP2 and RAMP3 in an animal immunized with said chimeric polypeptide., **characterized in that** the peptide from adrenomedullin has the sequence SEQ ID No. 2, the peptide from the CLR protein has the sequence SEQ ID No. 3 or SEQ ID No. 4, the peptide from the RAMP2 protein has the sequence SEQ ID No. 5 or SEQ ID No. 6, and the peptide from the RAMP3 protein has the sequence SEQ ID No. 7, SEQ ID No. 8 or SEQ ID No. 9.

2. A chimeric polypeptide capable of inducing the production of polyclonal antibodies which bind to adrenomedullin, CLR, RAMP2 and RAMP3 in an animal immunized with said chimeric polypeptide, **characterized in that** it presents the sequence SEQ ID No. 10.

3. The polypeptide according to any one of claim 1 or 2, for its use as a medicament.

4. The polypeptide for its use according to claims 3, for its use as a vaccine for the preventive or curative treatment of a disease in which angiogenesis must be inhibited, said disease being the cancer, the inflammatory diseases, the psoriasis, the atherosclerosis or the macular degeneration,

5. An immunogenic composition, **characterized in that** it comprises a chimeric polypeptide according to any one of claim 1 or 2 associated with at least one pharmaceutically acceptable vehicle and optionally at least one adjuvant.

6. A nucleic acid molecule, **characterized in that** it comprises a sequence coding for a chimeric polypeptide according to any one of claim 1 or 2.

7. An eukaryotic or prokaryotic recombinant vector, **characterized in that** it comprises an insert consisting of a nucleic acid molecule according to claim 6.

8. Eukaryotic or prokaryotic cells modified by a recombinant vector according to claim 7.


### SEQUENCE LISTING

<110> UNIVERSITE DE PROVENCE - AIX-MARSEILLE 1 MABROUK, Kamel OUAFIK, L'Houcine TESIC, Carine BERTIN, Denis BERENGUER-DAIZE, Caroline MARTIN, Pierre-Marie
<120> ANTICORPS SE LIANT A L'ADRENOMEDULLINE ET AUX RECEPTEURS DE L'ADRENOMEDULLINE ET LEURS UTILISATIONS COMME MEDICAMENT
<130> XRN/clg-F2245-1PCT
<150> FR 1003472
   <151> 2010-08-30
<160> 10
<170> PatentIn version 3.5
<210> 1
   <211> 52
   <212> PRT
   <213> Homo sapiens
<400> 1
   Tyr Arg Gln Ser Met Asn Asn Phe Gln Gly Leu Arg Ser Phe Gly Cys
   1               5                   10                  15
   Arg Phe Gly Thr Cys Thr Val Gln Lys Leu Ala His Gln Ile Tyr Gln
               20                  25                  30
   Phe Thr Asp Lys Asp Lys Asp Asn Val Ala Pro Arg Ser Lys Ile Ser
           35                  40                  45
   Pro Gln Gly Tyr
       50
<210> 2
   <211> 24
   <212> PRT
   <213> Artificial
<220>
   <223> Peptide dérivé de l'adrénomédulline
<400> 2
   Arg Ser Phe Arg Phe Gly Lys Leu Ala Thr Asp Lys Asp Lys Asp Asn
   1               5                   10                  15
   Val Ala Pro Arg Ser Lys Ile Ser
               20
<210> 3
   <211> 25
   <212> PRT
   <213> Artificial
<220>
   <223> Peptide dérivé de hCLR
<400> 3
   Ser Pro Glu Asp Ser Ile Gln Leu Gly Val Thr Arg Asn Lys Ile Met
   1               5                   10                  15
   Thr Ala Gln Tyr Glu Ala Tyr Gln Lys
               20                  25
<210> 4
   <211> 31
   <212> PRT
   <213> Artificial
<220>
   <223> Peptide dérivé de hCLR
<400> 4
   Pro Asp Tyr Phe Gln Asp Phe Asp Pro Ser Glu Lys Val Thr Lys Ile
   1               5                   10                  15
   Ala Asp Gln Asp Gly Asn Trp Phe Arg His Pro Ala Ser Asn Arg
               20                  25                  30
<210> 5
   <211> 23
   <212> PRT
   <213> Artificial
<220>
   <223> Peptide dérivé de hRAMP2
<400> 5
   Lys Asn Tyr Glu Thr Ala Val Gln Phe Ala Trp Asn His Tyr Lys Asp
   1               5                   10                  15
   Gln Met Asp Pro Ile Glu Lys
               20
<210> 6
   <211> 28
   <212> PRT
   <213> Artificial
<220>
   <223> Peptide dérivé de hRAMP2
<400> 6
   Arg Pro Tyr Ser Thr Leu Arg Asp Ala Leu Glu His Phe Ala Glu Leu
   1               5                   10                  15
   Phe Asp Leu Gly Phe Pro Asn Pro Leu Ala Glu Arg
               20                  25
<210> 7
   <211> 6
   <212> PRT
   <213> Artificial
<220>
   <223> Peptide dérivé de hRAMP3
<400> 7
   Lys Val Asp Val Trp Lys
   1               5
<210> 8
   <211> 22
   <212> PRT
   <213> Artificial
<220>
   <223> Peptide dérivé de hRAMP3
<400> 8
   Leu Glu Arg Leu Pro Leu Ala Gly Lys Ala Phe Ala Asp Met Met Gly
   1               5                   10                  15
   Lys Val Asp Val Trp Lys
               20
<210> 9
   <211> 22
   <212> PRT
   <213> Artificial
<220>
   <223> Peptide dérivé de hRAMP3
<400> 9
   Gly Phe Ile Thr Gly Ile His Arg Gln Phe Phe Ser Asn Ala Thr Val
   1               5                   10                  15
   Asp Arg Val His Leu Glu
               20
<210> 10
   <211> 78
   <212> PRT
   <213> Artificial
<220>
   <223> Polypeptide chimère
<400> 10
   Arg Ser Phe Arg Phe Gly Lys Leu Ala Thr Asp Lys Asp Lys Asp Asn
   1               5                   10                  15
   Val Ala Pro Arg Ser Lys Ile Ser Pro Asp Tyr Phe Gln Asp Phe Asp
               20                  25                  30
   Pro Ser Glu Lys Val Thr Lys Ile Ala Asp Gln Asp Gly Asn Trp Phe
           35                  40                  45
   Arg Lys Val Asp Val Trp Lys Lys Asn Tyr Glu Thr Ala Val Gln Phe
       50                  55                  60
   Ala Trp Asn His Tyr Lys Asp Gln Met Asp Pro Ile Glu Lys
   65                  70                  75